# EUROPEAN PATENT SPECIFICATION

(11) **EP 1 678 196 B1**
(45) Date of publication and mention of the grant of the patent: **13.08.2008**
(21) Application number: 04767097.1
(22) Date of filing: 05.10.2004
(51) Int. Cl.: C07K 5/06, A61K 38/05

(54) **SULFONYLAMINO-PEPTIDOMIMETICS ACTIVE ON THE SOMATOSTATIN RECEPTOR SUBTYPES 4 (SSTR4) AND 1 (SSTR1)**
SULFONYLAMINO-PEPTIDOMIMETIKA, DIE AUF DIE SOMATOSTATINREZEPTOR-SUBTYPEN 4 (SSTR4) UND 1 (SSTR1) WIRKEN
SULFONYLAMINO-PEPTIDOMIMETIQUES ACTIFS SUR LES SOUS-TYPES 4 (SSTR4) ET 1 (SSTR1) DU RECEPTEUR DE SOMATOSTATINE

(30) Priority: 06.10.2003 US 508270 P; 06.10.2003 FI 20031455
(43) Date of publication of application: 12.07.2006
(73) Proprietor: Wurster, Siegfried, 21500 Piikkiö (FI)
(72) Inventor: TOMPERI, Jussi, FI-20540 Turku (FI); ENGSTRÖM, Mia, FI-20500 Turku (FI); WURSTER, Siegfried, FI-21500 Piikkiö (FI)
(74) Representative: Syvänen, Ralf Ossian
(86) International application number: PCT/FI2004/000583
(87) International publication number: WO 2005/033124

(56) References cited:
- WO-A1-02/092566
- WO-A2-03/026575
- ROHRER S.P. ET AL: 'Rapid Identification of Subtype-Selective Agonists of the Somatostatin Receptor Through Combinatorial Chemistry' SCIENCE vol. 282, 23 October 1998, pages 737 - 740, XP002189092

## Description

### Field of the Invention

The present invention relates to 1-naphtalenesulfonylamino based peptidomimetics, which are useful for treating or diagnosing medical disorders related to somatostatin receptor subtype 4 optionally together with subtype 1.

### Background of the Invention

Somatostatin is a cyclic peptide found endogenously in two major forms made up of 14 (sst-14) or 28 (sst-28) amino acids. The shorter sst-14 is identical in sequence to the C-terminal half of sst-28. Somatostatin is produced widely in the body and acts both systemically and locally to inhibit the secretion of various hormones, growth factors and neurotransmitters. The biological effects of somatostatin are mediated by a family of G protein-coupled receptors, of which five subtypes (SSTR1-5) have been cloned in humans (Reisine and Bell 1995; Patel 1999). The affinities of the two endogenous forms of somatostatin on the five subtypes are relatively similar (sst-28 has been reported to have a moderate preference for the SSTR5). However, the five subtypes are differentially expressed in different tissues and do also show some differences in their interaction with a number of signalling pathways. Thus, the pleiotropic physiological responses mediated by somatostatin are a reflection of its widespread distribution and the existence of multiple receptor subtypes.

Based on their sequence similarity and their affinity towards a number of octapeptide and hexapeptide analogs to somatostatin, the family of five somatostatin receptor subtypes can be divided into two subfamilies: one subfamily made up of SSTR2, SSTR3 and SSTR5 and another subfamily made up of SSTR1 and SSTR4. The former possesses high and the latter rather low affinity towards the aforementioned hexapeptide and octapeptide analogs (Hoyer et al. 1995). Due to the availability of high affinity and selective ligands, the physiology of the SSTR2,3,5 subfamily has been more thoroughly characterized and it appears that the 'classical' effects of somatostatin, such as very potent inhibition of growth hormone, insulin, glucagon and gastric acid release, are mediated either primarily or exclusively via members of this subfamily.

Even though the physiology and pathophysiology of the subtypes SSTR1 and SSTR4 are less well understood, there have been a number of findings about the role of these subtypes described in scientific publications and the patenting literature. US6,124,256 reported that, given their localisation in the vascular wall and their time-related induction during the proliferative stage, SSTR1 and/or SSTR4 may be the optimal subtypes to prevent fibroproliferative vasculopathy via a somatostatin receptor based therapy. In agreement with this, Curtis et al. (2000) have described SSTR1 and SSTR4 to represent the predominant subtypes expressed in human blood vessels and have proposed the use of SSTR1- or SSTR4-selective agonists for the treatment of endothelial cell-mediated proliferative diseases. Aavik et al. (2002) have demonstrated that a purportedly SSTR1- and SSTR4-selective peptide analogue of somatostatin (CH-275) is able to prevent intimal hyperplasia after rat carotid denudation injury. Taken together, these findings may explain why two peptide analogues of somatostatin, octreotide and lanreotide, which possess very high preferences for the subtypes SSTR2 and SSTR5 but have rather low affinities for the subtypes SSTR1 or SSTR4, failed to show efficacy in clinical trials aiming at the prevention of restenosis after percutaneous transluminal angioplasty (Eriksen et al. 1995; van Essen et al. 1997).

Due to the fact that SSTR1 activation causes antiproliferative effects, SSTR1-selective agonist may be useful for the treatment of SSTR1 bearing tumors. For example, it has been described that SSTR1 receptors are expressed in prostate cancer (Sinisi et al. 1997, Reubi et al. 1997, Reubi et al. 2001) but not in normal prostate tissue. Independent of its functional properties as an agonists or an antagonist, any SSTR1 selective ligand may be useful for the diagnosis of prostate tumors or tumors in other tissues expressing the SSTR1 subtype.

WO97/03054 and US6,221,870 describe benzo[g]quinoline-derived (WO097/03054) or ergoline-derived (US6,221,870) SSTR1-selective antagonist as lowering aggressive behavior in mice and, based on this observation, suggest such compounds to be useful for the treatment of depression, anxiety, affective disorders and attention deficit and hyperactivity disorders.

According to Bito et al. (1994) the SSTR4 subtype is expressed at high levels in the rat hippocampus where somatostatin has been reported to play a significant role in the regulation of membrane conductance. Since the hippocampus is a brain structure closely linked to learning and memory, as well as mental disorders such as depression and schizophrenia, the prominent role of the SSTR4 subtype in the hippocampus suggests that SSTR4 selective agonists or antagonists with the ability to pass the blood-brain-barrier may have therapeutic potential.

Employing in situ hybridisation, Mori et al. (1997) have shown that in the rat eye SSTR4 expression predominates in the posterior iris epithelium and ciliary body. In addition, the authors have observed that somatostatin lowers intraocular pressure (iop) and, based on these observations, they have suggested that SSTR4-selective ligands may be useful as anti-glaucoma agents.

Somatostatin has a very short biological half-life and is therefore unsuitable for therapeutic use. A number of shorter hexa- and octapeptide analogs of somatostatin with improved biological stability have been identified (e.g. patents US4,485,101, US5,409,894 or WO97/47317). However, these abbreviated peptide analogs are heavily biased in favour of the SSTR2,3,5 subfamily and do not show any significant interaction with the subtypes SSTR1 or SSTR4. In contrast, WO97/14715 and Rivier et al. (2001) describe a group of SSTR1 preferring undecapeptide agonists. However, besides their often rather short biological half-lifes peptides also possess other problematic properties, which make them unsatisfactory as medicines. For example, peptides have a very limited ability to penetrate membranes. This is one of the reasons, why it is in most cases impossible to apply peptides via an oral route and why peptides generally do not reach the central nervous system.

In recent years, a number of nonpeptide somatostatin agonists have been identified. Besides the already mentioned SSTR1-selective antagonists reported in WO97/03054 and US6,221,870, WO97/43278 describes a number of thiourea-based compounds that preferentially interact with the somatostatin SSTR4 and the histamin H₃ subtype. US6,329,389 and US6,352,982 provide SSTR4-selective compounds centred around tetrahydroquinoline or 4,1-benzoxazepine scaffolds. Rohrer et al. (1998) have been able to identify subtype-selective agonists for each of the five somatostatin receptor subtypes by employing a combinatorial chemistry strategy which incorporated the generally accepted hypothesis on the structure-activity-relationship of somatostatin receptor active compounds that the amino acid residues 8 and 9 in sst-14 (which consist of a tryptophan and a lysine) are essential for proper ligand-receptor interaction.

The current invention describes a group of compounds from a new class of somatostatin ligands, 1-naphthalenesulfonamido-peptidomimetics. These compounds are in part related to sulfonamido-peptidomimetics, which have been presented in the context of another G-protein coupled receptor, the neuropeptide FF receptor. Sulfonylamino derivatives of monocyclic or bicyclic amino acids have also been described in US6,271,252 and US6,221,888 as cell adhesion molecule (CAM) antagonists which inhibit leukocyte adhesion and leukocyte adhesion-mediated pathologies.

Neuropeptide FF is an octapeptide originally isolated in 1985 by Yang et al. from bovine brain. It is named for the fact that both its N-terminal as well as its C-terminal consist of a phenylalanine, the single letter amino acid abbreviation of which is F. In the literature neuropetide FF has also been called F8Famide or morphine modulating peptide. Neuropeptide FF receptors are known to exist as two different subtypes called NPFF-1 and NPFF-2 (Bonini et al. 2000). Structure-activity-relationship (SAR) studies by Payza et al. (1993), Gicquel et al. (1994), Bourguignon et al. (1997) and Mazarguil et al. (2001) have investigated which of the structural motives in peptide-based NPFF receptor ligands are important in order to achieve proper interactions with NPFF receptor. In particular, it has been demonstrated that truncation of the four N-terminal amino acids in the octapeptide NPFF is compatible with maintaining good binding affinities. Within truncated tetrapeptides there is a relatively wide spectrum of acceptable amino acids for position one and two of the peptide sequence, provided the two C-terminal amino acids consist of an arginine (the single letter amino acid abbreviation of which is R) and an amidated phenylalanine (single letter F). In contrast, attempts to modify the C-terminal sequence by replacing either the arginine or the phenylalanine or by converting the amidated C-terminal into a free carboxylic acid invariably led to large losses in binding affinities. These observations identified a C-terminal 'RFamide' moiety as the most critical structural feature of peptidic or peptide-derived NPFF receptor ligands. In agreement with these SAR conclusions, WO02/24192 and WO03/026575 describe a number of RFamide-based or RFamide-related dipeptides, which are N-terminally extended by substituents that are connected either via an amide (WO02/24192) or via a sulfonamide bond (WO03/026575). The sulfonamido-peptidomimetics are an extension of dansyl-RFamide, a compound which has been introduced by Brussaard et al. (1989) as a tool to study the pharmacological effects of FMRFamide. Dansyl-RFamide has been shown to bind to NPFF receptor in rat tissue with an affinity of 73 nM (Payza et al. 1993).

### Summary of the Invention

It has now surprisingly been found that sulfonylamino-RFamide compounds, in particular 1-naphthalenesulfonyl-Arg-Phe-NH₂, do not only bind to NPFF receptor, but also, and with higher affinity than reported for NPFF receptor, to the somatostatin receptor subtypes SSTR1 and especially SSTR4. In contrast, the interaction with the somatostatin receptor subtypes SSTR2, SSTR3 and SSTR5 universally is of low affinity. By further exploring the structural requirements for the interaction with the two somatostatin receptor subtypes we have also discovered that, in contrast to the evidence available for NPFF receptor, the arginine is not an absolute requirement in order to obtain high affinity on the SSTR4 receptor. As a matter of fact, for this somatostatin receptor subtype the arginine can be replaced by a number of other amino acid based motives, provided these motives possess a basic side chain like arginine does.

Due to their high selectivity and affinity for SSTR4 and SSTR1 receptor, the compounds of the current invention may be used for a wide variety of therapeutic, prophylactic and diagnostic applications:
1. Compounds of the invention are useful for the prevention or treatment of diseases or symptoms of anxiety, depression, schizophrenia, epilepsy, attention deficit and hyperactive disorders and neurodegenerative diseases such as dementia, Alzheimer's disease and Parkinson's disease. The treatment of affective disorders includes bipolar disorders, e.g. manic-depressive psychoses, extreme psychotic states, e.g. mania, and excessive mood swings for which a behavioural stabilization is being sought. The treatment of anxiety states includes generalized anxiety as well as social anxiety, agoraphobia and those behavioural states characterized by social withdrawal, e.g. negative symptoms.
2. Compounds of the invention are advantageous in diseases involving pathological vascular proliferation, e.g. angiogenesis, restenosis, smooth muscle proliferation, endothelial cell proliferation and new blood vessel sprouting or conditions requiring the activation of neovascularization. The angiogenic disease may for example be age-related macular degeneration or vascular proliferation associated with surgical procedures, e.g. angioplasty and AV shunts. Other possible uses are the treatments of arteriosclerosis, plaque neovascularization, hypertrophic cardiomyopathy, myocardial angiogenesis, valvular disease, myocardiac infarction, coronary collaterals, cerebral collaterals and ischemic limb angiogenesis.
3. Compounds of the invention are also indicated for the treatment of diseases connected to pathological condition in the retina and/or iris-ciliary body of mammals. Such conditions may be high intraocular pressure (iop) and/or deep ocular infections. Treatable diseases may e.g. be glaucoma, stromal keratitis, iritis, retinitis, cataract and conjunctivitis. Other diseases connected to the eye may be ocular and corneal angiogenic conditions, for example, corneal graft rejection, retrolental fibroplasia, Osler-Webber Syndrome or rubeosis.
4. Compounds of the invention are also useful for the prevention or treatment of diseases or symptoms connected to diabetic complications such as diabetic retinopathy, diabetic nephropathy, diabetic neuropathy, Doan syndrome and orthostatic hypotension.
5. Compounds of the invention are useful for the treatment of a number of tumors such as e.g. the proliferation of adenoma cells, thyroid cancer, large bowel cancer, breast cancer, prostatic cancer, small cell lung cancer, non-small cell cancer, pancreatic cancer, stomach cancer, GI tumors, cholangiocarcinoma, hepatic cancer, vesical cancer, ovarian cancer, melanoma, osteosarcoma, chondrosarcoma, malignant pheochromocytoma, neuroblastoma, brain tumors, thymoma, paragangliomas, prostate carcinomas, sarcomas, gastroenteropancreatic tumors, gastric carcinomas, phaeochromocytomas, ependymomas, renal cancers, leukemia e.g., leukemla of basophilic leukemia, chronic lymphocytic leukemia, chronic myeloid leukemia, Hodgkln disease and non-Hodgkin lymphoma.
6. Compounds of the invention can also be used for the imaging of healthy or diseased tissues and/or organs (such as brain, blood vessels or tumors) possessing in particular SSTR4 receptors.
7. Compounds of the Invention are useful for targeting tumors with SSTR1 and/or SSTR4 receptors using a compound of the invention conjugated with anti-cancer drugs directly or using a suitable spacer.
8. Finally, compounds of the invention are useful for wound healing, peptic ulcers, psoriasis, rheumatoid arthritis and Crohn's disease.

### Detailed description of the invention

The invention relates to 1-naphthalenesulfonylamino based peptidomimetics having the following general formula (I) 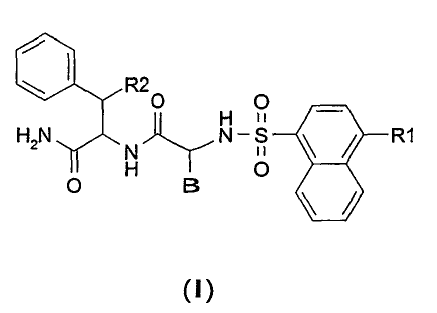 and pharmaceutically acceptable salts thereof;
wherein R1 is H, methyl or ethyl;
R2 is H or phenyl; and
B is
1) -(CH₂)₃NHC(NH)NH₂,
2) -(CH₂)₃NH₂,
3) -(CH₂)₂NH₂ or
4) 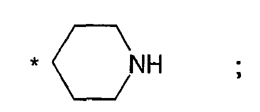
wherein asterisk (*) indicates the point of attachment;
with the provisio that when B is -(CH₂)₃NHC(NH)NH₂, then R1 is not hydrogen.

The compounds as well as the pharmaceutically acceptable salts thereof, are referred to below as the compounds of the invention, unless otherwise indicated.

Unless indicated by specific configurations at chiral centre, the invention includes within its scope all possible stereoisomers of a particular compound, including optical isomers, e.g. enantiomers. Furthermore, the invention includes in its scope both the individual isomers and any mixtures thereof, e.g. racemic mixtures. The individual isomers may be obtained using the corresponding isomeric forms of the starting material or they may be separated after the preparation of the end compound according to conventional separation methods. For the separation of optical isomers, e.g. enantiomers, from the mixture thereof the conventional resolution methods, e.g. fractional crystallisation, may be used.

Pharmaceutically acceptable salts, e.g. acid addition salts with both organic and inorganic acids, are well known in the field of pharmaceuticals. Non-limiting examples of these salts include chlorides, bromides, sulfates, nitrates, phosphates, sulfonates, formates, tartrates, maleates, citrates, benzoates, salicylates and ascorbates.

The pharmaceutical compositions of the compounds of the invention may be formulated in a conventional manner using one or more pharmaceutically acceptable carriers or excipients. Formulations can for instance enable for oral, buccal, topical, intranasal, parenteral (e.g. intravenous, intramuscular or subcutaneous) or rectal administration or administration by inhalation or insufflation. Compounds of the invention may also be formulated for sustained delivery.

For oral administration, forms of suitable compositions include but are not limited to tablets, chewable tablets and capsules. These may be prepared by conventional means with pharmaceutically acceptable excipients, such as binding agents (e.g. pregelatinized maize starch), disintegrants (e.g. potato starch), fillers (e.g. lactose) or lubricants (e.g. magnesium stearate). Tablets may be coated by methods well known in the art. For oral administration, possible liquid preparations include but are not limited to solutions, syrups or suspensions, or they may exist as dry powder for constitution with water or other suitable vehicle prior use. These liquid preparations may be prepared by conventional means with pharmaceutically acceptable agents, such as suspending agents, non-aqueous vehicles, preservatives and emulsifyiers.

A possible dose of the active compounds of the invention for oral, parenteral, buccal or topical dose to the adult human is between 0.1 and 500 mg of the active compound per unit dose, which may administered, for instance, 1 to 4 times in a day.

It is well recognized that the precise dose, the route of administration and the dosing interval can be determined by those skilled in the art. It is also well recognized that these variables depend on multiple factors including but not restricted to activity of the therapeutic compound, the formulation thereof, pharmacokinetic properties (such as absorption, distribution, metabolism and excretion) of the therapeutic compound, the nature and location of the target tissue or organ and the issues connected to the state of a disease or disorder in a patient in need of treatment. Additionally, when the compounds of the invention are administered with additional pharmaceutically active ingredients, one or more pharmaceutical compositions may be used for the delivery of all the agents, which may be administered together, or at different times, as determined by those skilled in the art.

The compounds of the current invention can be viewed as consisting of different motives: an 'aromatic part', a 'carboxylic acid' and a 'sulfonylamino' part. Thus, the compounds of the invention are named as amides wherein the 'carboxylic acid' forms the parent structure and is amidated by the 'aromatic part' and further substituted by the 'sulfonylamino' and an additional basic function. Naming is exemplified in the preferred embodiments below.

Especially preferred embodiments of the compounds of the invention are
● *N*-((*S*)-1-carbamoyl-2-phenylethyl)-5-guanidino-(*S*)-2-(*N*'-(4-methyl-1-naphthalenesulfonyl)amino)pentanamide (Compound 1) 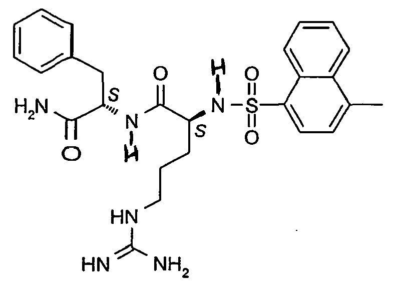
● *N*-((*S*)-1-carbamoyl-2,2-diphenylethyl)-5-guanidino-(*S*)-2-(*N'*-(4-methyl-1-naphthalenesulfonyl)amino)pentanamide (Compound 2) 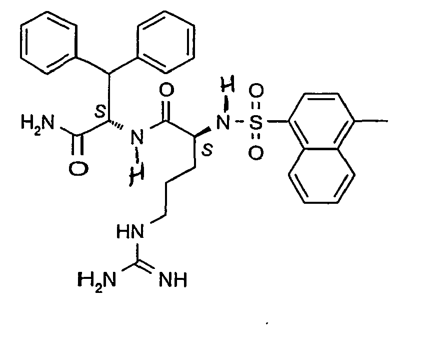
● 5-amino-*N*-((*S*)-1-carbamoyl-2-phenylethyl)-(*S*)-2-(*N'*-(4-methyl-1-naphthalenesulfonyl)amino)pentanamide (Compound 3) 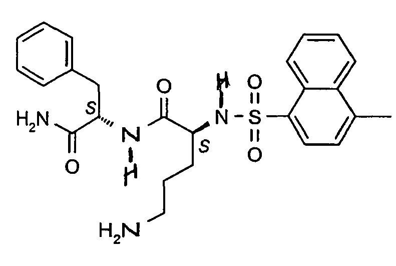
● 4-amino-*N*-((*S*)-1-carbamoyl-2-phenylethyl)-(S)-2-(*N'*-(1-naphthalenesulfonyl)amino)butanamide (Compound 4) 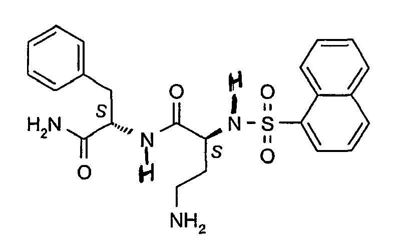
● 4-amino-*N*-((*S*)-1-carbamoyl-2-phenylethyl)-(*S*)-2-(*N'*-(4-methyl-1-naphthalenesulfonyl)amino)butanamide (Compound 5) 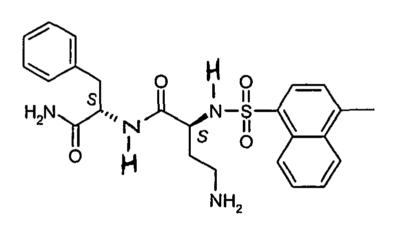
● 4-amino-*N*-((*S*)-1-carbamoyl-2-phenylethyl)-(*S*)-2-(*N'*-(4-ethyl-1-naphthalenesulfonyl)amino)butanamide (Compound 6) 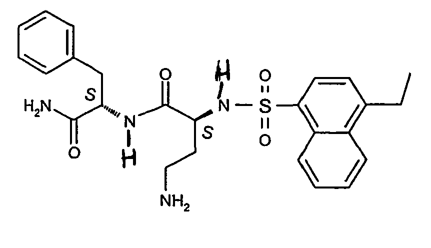
● 4-amino-*N*-((*S*)-1-carbamoyl-2,2-diphenylethyl)-(*S*)-2-(*N'*-(4-methyl-1-naphthalenesulfonyl)amino)butanamide (Compound 7) 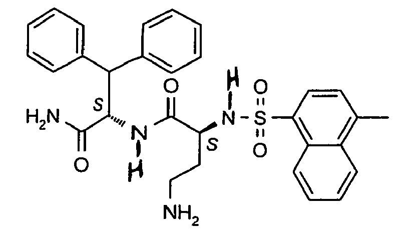
● *N*-((*S*)-1-carbamoyl-2-phenylethyl)-2-(*N'*-(4-methyl-1-naphthalenesulfonyl)amino)-2-pyridin-4-ylacetamide (Compound 8)

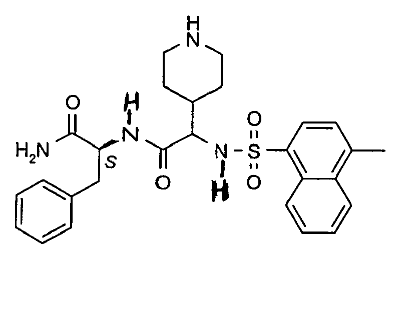

### Experimental part

### List of abbreviations:

- ACN: acetonitrile
- Boc: *tert*-butyloxycarbonyl
- BSA: bovine serum albumin
- DBU: 1,8-diazabicyclo[5.4.0]undec-7-ene
- Dbu: 2,4-diaminobutyric acid
- DCM: dichloromethane
- DIC: diisopropylcarbodiimide
- DIPEA: *N,N*-diisopropylethylamine
- DMF: *N,N*-dimethylformamide
- EDTA: ethylenediamine-tetraacetic acid
- ESI: electrospray ionization
- Fmoc: 9-fluorenylmethoxycarbonyl
- HEPES: *N*-(2-hydroxyethyl)piperazine-*N'*-2-ethanesulfonic acid
- HOBt: 1-hydroxybenzotriazole
- HPLC: high performance liquid chromatography
- LC: liquid chromatography
- MS: mass spectrometry
- PG: protecting group
- Pmc: 2,2,5,7,8-pentamethyl-chroman-6-sulphonyl
- RP-HPLC: reversed-phase high performance liquid chromatography
- TEA: triethylamine
- TFA: trifluoroacetic acid
- THF: tetrahydrofuran
- TLC: thin layer chromatography
- TMOF: trimethyl orthoformate
- TMS: tetramethylsilane
- TRIS: tris(hydroxymethyl)aminomethane

Compounds of the present invention may be prepared using the following general synthetic schemes.

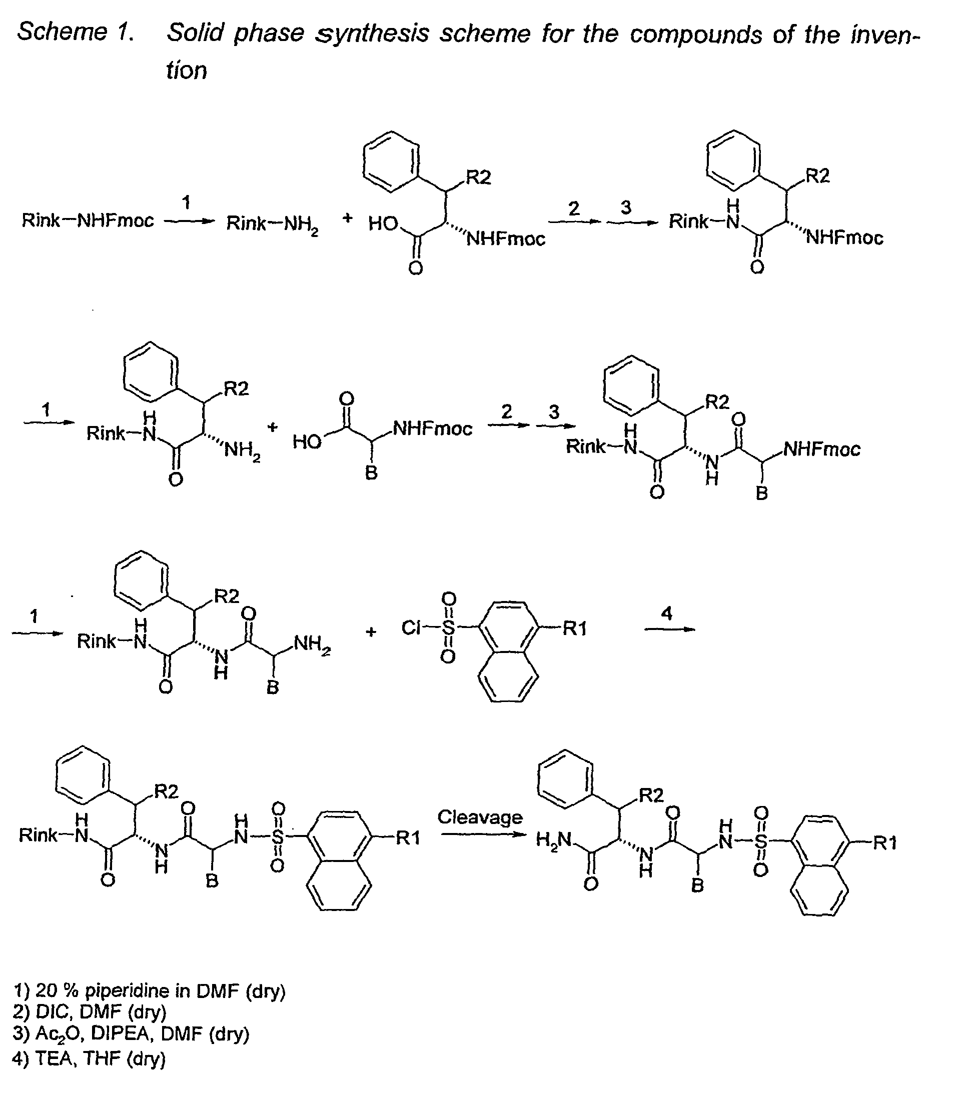

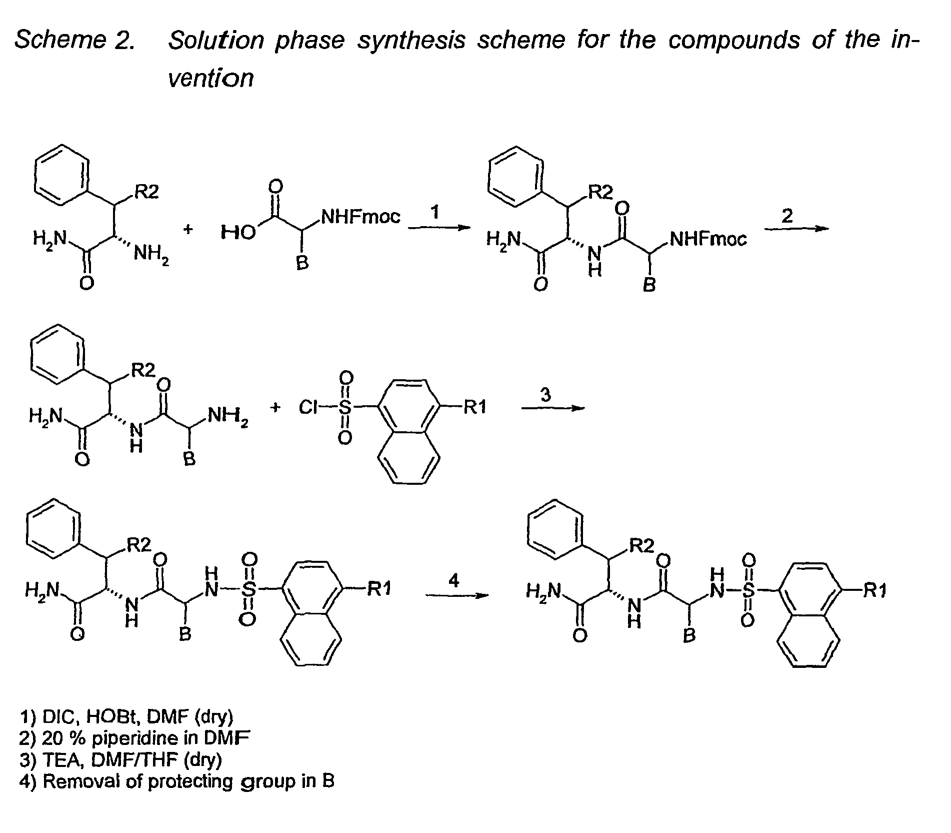

It is evident for a person skilled in the art that the general schemes can be further modified for example by using different protecting groups (e.g. those described in T.W. Greene and P.G.M. Wuts, "Protective Groups in Organic Synthesis", 2nd ed. Wiley, 1991, New York, US).

### Starting materials

The Rink amide resin was obtained from Advanced ChemTech, UK. Amino acids were purchased from either from Advanced ChemTech, UK or Novabiochem, Switzerland unless otherwise specified. DIC, HOBt, acetic anhydride and piperidine were products of Acros Organics, Belgium. DIPEA was from Fluka AG, Germany. All the other reagents or solvents were purchased from Aldrich or Merck, Germany, if not otherwise specified. The reagents were used as such and solvents were purified and dried according the methods described in W.L.F. Armareggo and D.D. Perrin, "Purification of Laboratory Chemicals", 4th ed. Butterworth-Heinemann, 1996, Bath, Great Britain.

### General description of MS analysis

Molecular weights of the compounds were determined with Micromass Micro triple quadrupole mass spectrometer. Essential MS parameters were: cone voltage 30 V, capillary voltage 3.5 kV, low mass resolution on MS1 15, high mass resolution on MS1 15, ion energy on MS1 1.0, source temperature 110°C, desolvation temperature 250°C and desolvation gas flow 700 l/h. Samples were introduced by Waters Alliance 2695 HPLC. Flow rate of 0.3 ml/min was formed of 10% water and 90% MeOH eluent (containing 0.01% HCOOH). Sample volume of 10 µl was injected through a Waters Symmetry Shield 2.1 X 10 mm C₁₈ precolumn.

### General description of LC-MS analysis

For LC-MS analysis the gradient started from 100% water (containing 0.01% HCOOH) (A) which changed linearly in ten minutes to 100% ACN (containing 0.01% HCOOH) (B). In addition, a Waters Symmetry Shield 2.1 X 50 mm C₁₈ column with a corresponding precolumn was flushed for two minutes with B. Flow rate was 0.4 ml/min and 10 µl of sample injected. Some essential MS parameters were increased compared to standard MS analysis: desolvation temperature to 350°C and desolvation gas flow to 900 l/h. UV chromatogram was recorded with Waters 996 diode array detector.

### General description of NMR analysis

NMR spectra were recorded on Bruker DMX 500 spectrometer operating at 500.13 MHz for ¹H. CD₃OD was used as a solvent and TMS as internal standard. If the final product consists of a mixture of diastereomers, only the signals corresponding to one of the isomers are given.

### General description of Flash Chromatography purification

Flash Chromatographic purification was conducted with Argonaut FlashMaster II Automated Purification System (Argonaut Technologies, UK) using normal phase columns (Supelco DSC-Si 20 g). Flow rate was 7 ml/min and detection wavelength 230 nm. Standard elution program was 25 minutes with the following gradient: 100% DCM for 3 minutes followed by gradual increase up to 25% MeOH during 17 minutes and a gradual increase up to 100% MeOH during the final 5 minutes. After MS verification, fractions containing the product were combined and evaporated.

### General description of RP-HPLC purification

Semi-preparative RP-HPLC purifications were done with Waters 616 pump, controlled by Waters 600 controller unit. Instrument was equipped with Waters 2487 UV detector and Waters fraction collector. Xterra Prep C₁₈ RP 10 X 150 mm column with 7.8 X 20 mm precolumn was used for purifications. Flow rate was 6.6 ml/min and the detection wavelength 254 nm. Gradient started with water (containing 0.3% HCOOH) (A) which changed linearly to ACN (containing 0.3% HCOOH) (B) within ten minutes. In addition, column was flushed with B for two minutes. Fraction collector was programmed to collect 30 s fractions. The fractions were analysed by MS.

### General description of LC purity analysis

HPLC purity of the compounds was determined using Waters 616 pump, controlled by Waters 600 controller unit. Instrument was further equipped with Waters 2487 UV detector (detection wavelengths 254 nm and 220 nm). Waters Symmetry Shield 2.1 X 50 mm C₁₈ column with corresponding precolumn and a flow rate of 0.4 ml/min was used. Linear gradient starting from water (containing 0.01% HCOOH) (A) to ACN (containing 0.01% HCOOH) (B) over 17 minutes and then 100 % B for 1 minute was applied.

### Example 1

### Synthesis of N-((S)-1-carbamoyl-2-phenylethyl)-5-guanidino-(S)-2-(N'-(4-methyl-1-naphthalenesulfonyl)amino)pentanamide (Compound 1)

### Step I

Rink amide resin (1 g, 0.7 mmol/g, 0.7 mmol) was washed twice with DMF prior use. Washed resin was dissolved in 12.5 ml of 20 vol-% piperidine in DMF and mixture was agitated for 35 minutes. Resin was then washed thrice with DMF, thrice with MeOH, twice with DCM and finally twice with THF. Resin was used immediately for step II.

### Step II

Fmoc-Phe-OH (813.6 mg, 387.44 g/mol, 2.1 mmol, 3 eq) and DIC (328.8 µl, 126.20 g/mol, 0.806 g/cm³, 2.1 mmol, 3 eq) were dissolved in dry DMF (12.5 ml) and after 10 minutes mixed with the resin. After 18 hours agitation, solvent was filtered out and fresh solution with half of the original amounts of Fmoc-Phe-OH and DIC in dry DMF was introduced. After additional 5.5 hours, solvent was again filtered out and resin washed thrice with DMF, thrice with MeOH, thrice with DCM and thrice with THF.

### Step III

Possibly unreacted amino groups of the resin were acetylated with a solution consisting of acetic anhydride (1 ml, 102.09 g/mol, 1.087 g/cm³, 10.6 mmol) and DIPEA (250 µl, 129.25 g/mol, 0.755 g/cm³, 1.46 mmol) in dry DMF (12 ml) for 45 minutes. Resin was then filtered and washed thrice with DMF, thrice with MeOH, twice with DCM and twice with THF.

### Step IV

Fmoc protection of the attached phenylalanine was removed according to procedure described in step I but without any washes prior treatment with piperidine/DMF.

### Step V

Fmoc-Arg(Pmc)-OH (928.0 mg, 662.8 g/mol, 1.4 mmol, 2 eq) was coupled to resin bou nd compound using the same coupling agent and procedure as described in step II.

### Step VI

Possibly unreacted amino groups of phenylalanine were acetylated using the procedure described in step III.

### Step VII

Fmoc protection of the arginine attached in step V was removed according to procedure described in step I but again without any washes prior treatment with piperidine/DMF.

### Step VIII

4-Methyl-1-naphthalenesulfonyl chloride (337.0 mg, 240.71 g/mol, 1.4 mmol, 2 eq, Maybridge) was dissolved in dry THF (12.5 ml) and mixed with the resin. TEA (194.1 µl, 101.19 g/mol, 0.73 g/cm³, 1.4 mmol, 2 eq, Baker) was then added to the mixture. After overnight agitation, solvent was filtered and resin washed thrice with THF, thrice with MeOH, thrice with DMF, once with MeOH and finally thrice with DCM.

### Step IX

Resin bound product was cleaved and Pmc protection removed by treating the resin with 50 vol-% TFA in DCM (12.5 ml) for 1 hour. Resulting red solution was collected and evaporated. 116.5 mg of *N*-((*S*)-1-carbamoyl-2-phenylethyl)-5-gua nidino-(S)-2-(N'-(4-methylnaphthalene-1-sulfonyl)amino)-pentanamide as a dark oil was obtained. Product was purified using flash chromatography to give 50.8 mg of *N*-((*S*)-1-carbamoyl-2-phenylethyl)-5-guanidino-(*S*)-2-(*N'*-(4-methyl-1-naphthalenesulfonyl)amino)pentanamide as white solid, overall yield 14 %.
MS-ESI⁺ (m/z): 525
¹H NMR (500 MHz, CD₃OD; δ, ppm): 8.79 (m, 1 H), 8.23 (m, 1 H), 8.14 (d, 1 H), 7.76 (m, 2H), 7.47 (m, 1 H), 7.33-7.18 (m, 5H), 4.39 (m, 1 H), 3.62 (m, 1 H), 3.03 (m, 1 H), 2.94-2.78 (m, 5H), 2.68 (m, 1 H), 1.50 (m, 2H), 1.35 (m, 1H), 1.21 (m, 1H).

### Example 2

### Synthesis of N-((S)-1-carbamoyl-2,2-diphenylethyl)-5-guanidino-(S)2-(N'-(4-methyl-1-naphthalenesulfonyl)amino)pentanamide (Compound 2)

### Step I

Rink amide resin (1.45 g, 0.7 mmol/g, 1.02 mmol) was washed twice with DMF prior use. Washed resin was dissolved in 21 ml of 20 vol-% piperidine in DMF and mixture was agitated for 50 minutes. Resin was then washed thrice with DMF, thrice with MeOH, twice with DCM and finally twice with THF. Resin was used immediately for step II.

### Step II

Fmoc-L-3,3-diphenylalanine (1.41 g, 463.53 g/mol, 3.05 mmol, 3 eq, PepTech) and DIC (477.3 µl, 126.20 g/mol, 0.806 g/cm³, 3.05 mmol, 3 eq) were dissolved in dry DMF (21 ml) and after 10 minutes mixed with the resin. After 22 hours agitation, solvent was filtered out and fresh solution with similar amounts of Fmoc-L-3,3-diphenylalanine and DIC in dry DMF was introduced. After additional 5 hours, solvent was again filtered out and resin washed thrice with DMF, thrice with MeOH, thrice with DCM and thrice with THF.

### Step III

Possibly unreacted amino groups of the resin were acetylated with a solution consisting of acetic anhydride (700 µl, 102.09 g/mol, 1.087 g/cm³, 7.5 mmol) and DIPEA (119 µl, 129.25 g/mol, 0.755 g/cm³, 0.7 mmol) in dry DMF (16.1 ml) for 45 minutes. Resin was then filtered and washed thrice with DMF, thrice with MeOH, twice with DCM and twice with THF.

### Step IV

Fmoc protection of the attached 3,3-diphenylalanine was removed according to proced ure described in step I but without any washes prior treatment with piperidine/DMF.

### Step V

Fmoc-Arg(Pmc)-OH (1.34 g, 662.8 g/mol, 2.03 mmol, 2 eq) was coupled to resin bound compound using the same coupling agent and procedure as described in step II.

### Step VI

Possibly unreacted amino groups of 3,3-diphenylalanine were acetylated using the procedure described in step III.

### Step VII

Fmoc protection of the arginine attached in step V was removed according to procedure described in step I but again without any washes prior treatment with piperidine/DMF.

### Step VIII

4-Methyl-1-naphthalenesulfonyl chloride (733.7 mg, 240.71 g/mol, 3.0 mmol, 3 eq, Maybridge) was dissolved in dry THF (21 ml) and mixed with the resin. TEA (422.5 µl, 101.19 g/mol, 0.73 g/cm³, 3.0 mmol, 3 eq, Baker) was then added to the mixture. After overnight agitation, solvent was filtered and resin washed thrice with THF, thrice with MeOH, thrice with DMF, once with MeOH and finally thrice with DCM.

### Step IX

Resin bound product was cleaved and Pmc protection removed by treating the resin with 50 vol-% TFA in DCM (21 ml) for 1 hour. Resulting red solution was collected and evaporated. Product was purified with RP-HPLC to give 108.4 mg of *N*-((*S*)-1-carbamoyl-2,2-diphenylethyl)-5-guanidino-(*S*)-2-(*N'-*(4-methyl-1-naphthalenesulfonyl)amino)pentanamide as white solid, overall yield 16.4%.
MS-ESI⁺ (m/z): 601
¹H NMR (500 MHz, CD₃OD; δ, ppm): 8.72-8.69 (m, 1H), 8.19-8.16 (m, 1 H), 7.94-7.93 (m, 1 H), 7.69-7.65 (m, 2H), 7.36-7.34 (m, 1 H), 7.27-7.19 (m, 9H), 7.17-7.13 (m, 2H), 5.12-5.10 (m, 1H), 4.39-4.37 (d, 1H), 3.48-3.45 (m, 1 H), 2.77 (s, 3H), 2.76-2.65 (m, 2H), 1.44-1.02 (m, 4H).

### Example 3

### Synthesis of 5-amino-N-((S)-1-carbamoyl-2-phenylethyl)-(S)-2-(N'-(4-methyl-1-naphthalenesulfonyl)amino)pentanamide (Compound 3)

### Step I

Rink amide resin (30.0 mg, 0.7 mmol/g, 0.021 mmol) was washed twice with DMF prior use. Washed resin was dissolved in 2.5 ml of 20 vol-% piperidine in DMF and mixture was agitated for 50 minutes. Resin was then washed thrice with DMF, thrice with MeOH, twice with DCM and finally twice with THF. Resin was used immediately for step II.

### Step II

Fmoc-Phe-OH (24.4 mg, 387.44 g/mol, 0.063 mmol, 3 eq) and DIC (9.9 µl, 126.20 g/mol, 0.806 g/cm³, 0.063 mmol, 3 eq) were dissolved in dry DMF (2.5 ml) and after 10 minutes mixed with the resin. After 22 hours, solvent was filtered out and fresh solution with similar amounts of Fmoc-Phe-OH and DIC in dry DMF was introduced. After additional 5 hours, solvent was again filtered out and resin washed thrice with DMF, thrice with MeOH, thrice with DCM and thrice with THF.

### Step III

Possibly unreacted amino groups of the resin were acetylated with a solution consisting of acetic anhydride (100 µl, 102.09 g/mol, 1.087 g/cm³, 1.06 mmol) and DIPEA (17 µl, 129.25 g/mol, 0.755 g/cm³, 0.1 mmol) in dry DMF (2.1 ml) for 45 minutes. Resin was then filtered and washed thrice with DMF, thrice with MeOH, twice with DCM and twice with THF.

### Step IV

Fmoc protection of the attached phenylalanine was removed according to procedure described in step I but without any washes prior treatment with piperidine/DMF.

### Step V

Fmoc-Orn(Boc)-OH (28.6 mg, 454.5 g/mol, 0.063 mmol, 3 eq) was coupled to resin bound compound using the same coupling agent and procedure as described in step II.

### Step VI

Possibly unreacted amino groups of phenylalanine were acetylated using the procedure described in step III.

### Step VII

Fmoc protection of the ornithine attached in step V was removed according to procedure described in step I but again without any washes prior treatment with piperidine/DMF.

### Step VIII

4-Methyl-1-naphthalenesulfonyl chloride (15.2 mg, 240.71 g/mol, 0.063 mmol, 3 eq, Maybridge) was dissolved in dry THF (2.5 ml) and mixed with the resin. TEA (8.7 µl, 101.19 g/mol, 0.73 g/cm³, 0.063 mmol, 3 eq, Baker) was then add ed to the mixture. After overnight agitation, solvent was filtered and resin washed thrice with THF, thrice with MeOH, thrice with DMF, once with MeOH and finally thrice with DCM.

### Step IX

Resin bound product was cleaved and Boc protection removed by treating the resin with 25 vol-% TFA in DCM (2.5 ml) for 30 minutes. Resulting red solution was collected and evaporated. 11.0 mg of 5-amino-*N*-((*S*)-1-carbamoyl-2-phenylethyl)-(*S*)-2-(*N'*-(4-methyl-1-naphthalenesulfonyl)amino)-pentanamide as a dark oil was obtained; overall yield 88 %.
MS-ESI⁺ (m/z): 483

### Example 4

### Synthesis of 4-amino-N-((S)-1-carbamoyl-2-phenylethyl)-(S)-2-(N'-(1-naphthalenesulfonyl)amino)butanamide (Compound 4)

Compound was synthesised using the procedure described in example 3 but substituting the Fmoc-Orn(Boc) in step V with Fmoc-Dbu(Boc)-OH (27.8 mg, 440.48 g/mol, 0.063 mmol, 3 eq) and the 4-methyl-1-naphthalenesulfonyl chloride in step VIII with 1-naphthalenesulfonyl chloride (14.3 mg, 226.68 g/mol, 0.063 mmol, 3 eq, Acros). After cleavage 9.8 mg of 4-amino-*N*-((*S*)-1-carbamoyl-2-phenylethyl)-(*S*)-2-(*N'*-(1-naphthalenesulfonyl)amino)butanamide as a dark oil was obtained; overall yield 82 %.
MS-ES I⁺ (m/z): 455

### Example 5

### Synthesis of 4-amino-N-((S)-1-carbamoyl-2-phenylethyl)-(S)-2-(N'-(4-methyl-1-naphthalenesulfonyl)amino)butanamide (Compound 5)

### Step I

H-Phe-NH₂ hydrochloride (114.2 mg, 200.7 g/mol, 0.57 mmol, 1 eq, Advanced ChemTech) was dissolved in 2 ml of dry DMF/DCM (1/1) and TEA (95 µl, 101.19 g/mol, 0.73 g/cm³, 0.68 mmol, 1.2 eq) was added. After 30 minutes, a DMF/DCM (1/1, 4 ml) solution containing Fmoc-Dbu(Boc)-OH (250.2 mg, 440.5 g/mol, 0.57 mmol, 1 eq), DIC (89 µl, 126.20 g/mol, 0.805 g/cm³, 0.57 mmol, 1 eq) and HOBt (77.6 mg, 135.12 g/mol, 0.57 mmol, 1 eq) was added. After overnight stirring, solvent was evaporated and DCM added. Organic phase was washed thrice with water and once with brine. Part of the product precipitated from the water phase and after filtration it was combined with the evaporated organic phase. 333 mg of 4-(*N*-Boc-amino)-*N'*-((*S*)-1-carbamoyl-2-phenylethyl)-(S)-2-(N"-Fmoc-amino)butanamide was obtained as a white powder and with quantitative yield.

### Step II

Fmoc protection was removed by treating the 4-(*N*-Boc-amino)-*N'-*((*S*)-1-carbamoyl-2-phenylethyl)-(*S*)-2-(N*"*-Fmoc-amino)butanamide with 4.5 ml of 20 vol-% piperidine in DMF for 45 minutes. Solvent was then evaporated to give (*S*)-2-amino-4-(*N*-Boc-amino)-*N'*-((*S*)-1-carbamoyl-2-phenylethyl)butanamide as a white solid.

### Step III

Residue from step II was dissolved in 9 ml of dry THF/DMF (1/1) solution and 4-methyl-1-naphthalenesulfonylchloride (205.3 mg, 240.71 g/mol, 0.85 mmol, 1.5 eq, Maybridge) and finally TEA (120 µl, 101.19 g/mol, 0.73 g/cm³, 0.85 mmol, 1.5 eq) were added. After overnight reaction, solvent was evaporated and the residue purified with silica column chromatography (mobile phase from 5 % MeOH in DCM up to 20 % MeOH in DCM). 238 mg of 4-(*N-*Boc-amino)-*N'*-((*S*)-1-carbamoyl-2-phenylethyl)-(*S*)-2-(*N"*-(4-methyl-1-naphthalenesulfonyl)amino)butanamide as a white powder was obtained; yield 75%.

### Step IV

Boc protection was removed by dissolving the product from step III in 2.5 ml of 25 vol-% TFA in DCM and stirring for 1 h. Solvent was then evaporated and residue purified with RP-HPLC to give 52.5 mg of 4-amino-*N*-((*S*)-1-carbamoyl-2-phenylethyl)-(*S*)-2-(*N'*-(4-methyl-1-naphthalenesulfonyl)amino)-butanamide; yield 26.8%.
MS-ESI⁺ (m/z): 469
¹H NMR (500 MHz, CD₃OD; δ, ppm): 8.69 (m, 1 H), 8.16 (m, 1 H), 8.07 (m, 1 H), 7.69 (m, 2H), 7.39 (d, 1 H), 7.25-7.16 (m, 3H), 7.06 (m, 2H), 4.21 (t, 1 H), 3.84 (m, 1 H), 2.84-2.69 (m, 6H), 2.49 (m, 1 H), 1.94-1.74 (m, 2H).

### Example 6

### Synthesis of 4-amino-N-((S)-1-carbamoyl-2-phenylethyl)-(S)-2-(N'-(4-ethyl-1-naphthalenesulfonyl)amino)butanamide (Compound 6)

### Step I

Rink amide resin (50.0 mg, 0.7 mmol/g, 0.035 mmol) was washed twice with DMF prior use. Washed resin was dissolved in 2.5 ml of 20 vol-% piperidine in DMF and mixture was agitated for 50 minutes. Resin was then washed thrice with DMF, thrice with MeOH, twice with DCM and finally twice with THF. Resin was used immediately for step II.

### Step II

Fmoc-Phe-OH (40.7 mg, 387.44 g/mol, 0.105 mmol, 3 eq) and DIC (16.4 µl, 126.20 g/mol, 0.806 g/cm³, 0.105 mmol, 3 eq) were dissolved in dry DMF (2 ml) and after 10 minutes mixed with the resin. After overnight agitation, solvent was filtered out and resin washed thrice with DMF, thrice with MeOH, thrice with DCM and once with THF.

### Step III

Possibly unreacted amino groups of the resin were acetylated with a solution consisting of acetic anhydride (100 µl, 102.09 g/mol, 1.087 g/cm³, 1.06 mmol) and DIPEA (17 µl, 129.25 g/mol, 0.755 g/cm³, 0.1 mmol) in dry DMF (2.1 ml) for 45 minutes. Resin was then filtered and washed thrice with DMF, thrice with MeOH, twice with DCM and twice with THF.

### Step IV

Fmoc protection of the attached phenylalanine was removed according to procedure described in step I but without any washes prior treatment with piperidine/DMF.

### Step V

Fmoc-Dbu(Boc)-OH (40.3 mg, 440.5 g/mol, 0.091 mmol, 2.6 eq) was coupled to resin bound compound using the same coupling agent and procedure as described in step II.

### Step VI

Possibly unreacted amino groups of the phenylalanine were acetylated using the procedure described in step III.

### Step VII

N-alpha-Fmoc protection of the amino acid attached in step V was removed according to procedure described in step I but again without any washes prior treatment with piperidine/DMF.

### Step VIII

1-Ethylnaphthalene (1 ml, 156.23 g/ml, 1.008 g/cm³, 6.5 mmol, Aldrich) was mixed with 3 ml of TFA and mixture was cooled in water-ice bath. Chlorosulfonic acid (2 ml, 116.52 g/ml, 1.753 g/cm³, 30.1 mmol, 4.6 eq, Acros) was added dropwise to the mixture. During the addition, colour of the solution changed from red to dark. After addition, mixture was let to warm to ambient temperature. Reaction mixture was then transferred dropwise to vessel containing 40 ml of water in ice. Precipitate was filtered and washed twice with cold water. 0.46 g of 4-ethyl-1-naphthalenesulfonyl chloride as a white powder was obtained, yield 28%.

### Step IX

4-Ethyl-1-naphthalenesulfonyl chloride (44.0 mg, 254.74 g/mol, 0.17 mmol, 5 eq) was dissolved in dry THF (2.5 ml) and mixed with the resin. TEA (24 µl, 101.19 g/mol, 0.73 g/cm³, 0.17 mmol, 5 eq, Baker) was then added to the mixture. After overnight agitation, solvent was filtered and resin washed thrice with THF, thrice with MeOH, thrice with DMF, once with MeOH and finally thrice with DCM.

### Step X

Resin bound product was cleaved and Boc protection removed by treating the resin with 25 vol-% TFA in DCM (2.5 ml) for 1 hour. Resulting red solution was collected and evaporated. 12.5 mg of 4-amino-*N*-((*S*)-1-carbamoyl-2-phenylethyl)-(*S*)-2-(*N'*-(4-ethyl-1-naphthalenesulfonyl)amino)-butanamide as dark oil was obtained; overall yield 60%.
MS-ESI⁺ (m/z): 483

### Example 7

### Synthesis of 4-amino-N-((S)-1-carbamoyl-2,2-diphenylethyl)-(S)-2-(N'-(4-methyl-1-naphthalenesulfonyl)amino)butanamide (Compound 7)

Compound was synthesised using the procedure described in example 3 with following modifications. Fmoc-Phe-OH in step II was substituted with Fmoc-L-3,3-diphenylalanine-OH (29.2 mg, 463.53 g/mol, 0.063 mmol, 3 eq, PepTech), Fmoc-Orn(Boc) in step V was substituted with Fmoc-Dbu(Boc)-OH (27.8 mg, 440.48 g/mol, 0.063 mmol, 3 eq) and only 2 eq. of 4-methyl-1-naphthalenesulfonyl chloride (10.1 mg, 240.71 g/mol, 0.042 mmol, Maybridge) was used in step VIII. After cleavage, 10.3 mg of 4-amino-*N*-((*S*)-1-carbamoyl-2,2-d iphenylethyl)-(*S*)-2-(*N'*-(4-methyl-1-naphthalenesulfonyl)amino)butanamide as a dark oil was obtained; overall yield 74%.
MS-ESI⁺ (m/z): 545

### Example 8

### Synthesis of N-((S)-1-carbamoyl-2-phenylethyl)-2-(N'-(4-methy)-1-naphthalenesulfonyl)amino)-2-pyridin-4-ylacetamide (Compound 8)

Compound was synthesised using the procedure described in example 3 but Fmoc-Orn(Boc)-OH in step V was substituted with Fmoc- D,L-glycine(4-Boc-piperidinyl)-OH (20.2 mg, 480.6 g/mol, 0.042 mmol, 2 eq) and only 2 eq of it was used. After cleavage 12.0 mg of *N*-((*S*)-1-carbamoyl-2-phenylethyl)-2-(*N'*-(4-methyl-1-naphthalenesulfonyl)amino)-2-pyridin-4-ylacetamide as dark oil was obtained; overall yield 91 %.
MS-ESI⁺ (m/z): 509

### Example 9

### Binding affinity at the human somatostatin receptor subtypes

The affinity of the compounds of the invention for the five human somatostatin receptor subtypes (SSTR1, SSTR2, SSTR3, SSTR4, and SSTR5) was determined in competition binding assays with (¹²⁵I-Tyr)-[Leu⁸,DTrp²²]-somatostatin-28 (¹²⁵I-LTT-sst-28). The biological material for these experiments consisted of membranes from Chinese hamster ovary (CHO) cells stably transfected with one of the five human somatostatin receptor subtypes. Membranes (3-20 µg of total protein per sample) and trace amount of ¹²⁵I-LTT-sst-28 were incubated in 10 mM Hepes, 1 mM EDTA, 5 mM MgCl₂, 5 mg/ml of BSA and 30 µg/ml bacitracin, pH 7.6 with six concentrations of the compounds. Each concentration was run in duplicate. Nonspecific binding was defined by 1 µM somatostatin-14 (sst-14) and corresponded to 5-25% of total binding. After 60 min at room temperature, incubations were terminated by rapid vacuum filtration through GF/B glass fiber filter mats (presoaked at 4°C in 200 ml of 10 mM Hepes, 1 mM EDTA, 5 mM MgCl₂, pH 7.6) and three 5 ml washes with ice-cold wash buffer (20 mM TRIS, 1 mM EDTA, 5 mM MgCl_{2,} pH 7.4). The filters were then dried, impregnated with scintillate and their radioactivity was measured by scintillation counting. The analysis of the experiments was carried out by nonlinear least square curve fitting. Affinity constants (Kᵢ) were calculated from the IC₅₀ values according to the Cheng-Prusoff's equation (Cheng and Prusoff, 1973). Experiments were repeated a minimum of three times.

Using the aforementioned protocol, the following test results were obtained.

| compound | Kᵢ (SSTR1) / nM | Kᵢ (SSTR2) / nM | Kᵢ (SSTR3) / nM | Kᵢ (SSTR4) / nM | K¡ (SSTR5) / nM |
|---|---|---|---|---|---|
| compound 1 | 73 ± 19 | >10 000 | >10 000 | 3.6 ± 0.7 | >10 000 |
| compound 2 | 34 ± 14 | >10 000 | >5 000 | 1.5 ± 0.7 | >5 000 |
| compound 3 | 260 ± 20 | >1 000 | >1 000 | 6.5 ± 1.7 | >1 000 |
| compound 4 | >3 000 | >30 000 | >10 000 | 5.9 ± 2.9 | 6 600 ± 400 |
| compound 5 | 500 ± 150 | >1 000 | 1 400 ± 100 | 1.2 ± 0.4 | 540 ± 80 |
| compound 6 | 990 ± 80 | >10 000 | 2 900 ± 800 | 2.3 ± 0.4 | 2 300 ± 700 |
| compound 7 | >1 000 | >10 000 | >5 000 | 5.3 ± 2.1 | >10 000 |
| compound 8 | >1 000 | >10 000 | >10 000 | 3.2 ± 0.4 | >10 000 |

### References

■ Aavik et al. (2002), Elimination of vascular fibrointimal hyperplasia by somatostatin receptor 1,4-selective agonist, FASEB J 16:724-726
■ Bito et al. (1994), Functional coupling of SSTR4, a major hippocampal somatostatin receptor, to adenylate cyclase inhibition, arachidonate release and activation of the mitogen-activated protein kinase cascade, J Biol Chem 269:12722-12730
■ Bonini et al. (2000), Identification and characterization of two G protein-coupled receptors for NPFF, J Biol Chem 275:39324-39331
■ Bourguignon et al. (1997), Analogs of NPFF, a neuropeptide which modulates morphine analgesia, Proceedings of the XIVth International Symposium on Medicinal Chemistry, Awouters F (ed.), Elsevier Science B.V., pp. 35-44
■ Brussaard et al. (1989), Peripheral injection of DNS-RFa, a FMRFa agonist, suppresses morphine-induced analgesia in rats, Peptides 10:735-739
■ Cheng and Prusoff (1973), Relationship between the inhibition constant (KI) and the concentration of inhibitor which causes 50 per cent inhibition (I50) of an enzymatic reaction, Biochem Pharmacol 22:3099-3108
■ Curtis et al. (2000), Somatostatin receptor subtype expression and function in human vascular tissue, Am J Physiol Heart Circ Physiol 278:H1815-1822
■ Eriksen et al. (1995), Randomized double-blind Scandinavian trial of angiopeptin versus placebo for the prevention of clinical events and restenosis after coronary balloon angioplasty, Am Heart J 130:1-8
■ Gicquel et al. (1994), Structure-activity study of neuropeptide FF: contribution of N-terminal regions to affinity and activity, J Med Chem 37:3477-3481
■ Hoyer et al. (1995), Classification and nomenclature of somatostatin receptors, TIPS 16:86-88
■ Mazarguil et al. (2001), Structure-activity relationships of neuropeptide FF: role of C-terminal regions, Peptides 22:1471-1478
■ Mori et al. (1997), Differential expression of somatostatin receptors in the rat eye: SSTR4 is intensely expressed in the iris/ciliary body, Neurosci Lett 223:185-188
■ Patel (1999), Somatostatin and its receptor family, Front Neuroendocrinol 20:157-198
■ Payza et al. (1993), Neuropeptide FF receptors: structure-activity relationship and effect of morphine, J Pharm Exp Ther 267:88-94
■ Reisine and Bell (1995), Molecular biology of somatostatin receptors, Endocrinological Reviews 16:427-442
■ Reubi et al. (1997), A selective analog for the somatostatin sst1-receptor subtype expressed by human tumors, Eur J Pharmacol 345:103-110
■ Reubi et al. (2001), Somatostatin receptor sst1-sst5 expression in normal and neoplastic human tissues using receptor autoradiography with subtype-selective ligands, Eur J Nucl Med 28:836-846
■ Rivier et al. (2001), Potent somatostatin undecapeptide agonists selective for somatostatin receptor 1 (sst1), J Med Chem 44:2238-2246
■ Rohrer et al. (1998), Rapid identification of subtype-selective agonists of the somatostatin receptor through combinatorial chemistry, Science 282:737-740
■ Sinisi et al. (1997), Different expression patterns of somatostatin receptor subtypes in cultured epithelial cells from human normal prostate and prostate cancer, J Clin Endocrinol Metab 82:2566-2569
■ van Essen et al. (1997), Effects of octreotide treatment on restenosis after coronary angioplasty: results of the VERAS study, Circulation 96:1482-1487
■ Yang et al. (1985), Isolation, sequencing, synthesis and pharmacological characterisation of two brain neuropeptides that modulate the action of morphine, Proc Natl Acad Sci 82:7757-7781

## Claims

1. A compound of formula I 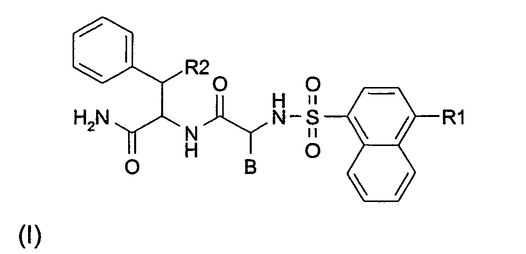 or a pharmaceutically acceptable salt thereof;
wherein R1 is H, methyl or ethyl; R2 is H or phenyl and
B is
1) -(CH₂)₃NHC(NH)NH₂,
2) -(CH₂)₃NH₂,
3) -(CH₂)₂NH₂ or
4) 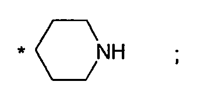
wherein asterisk (*) indicates the point of attachment;
with the proviso that when B is -(CH₂)₃NHC(NH)NH₂, then R1 is not hydrogen.

2. A compound of claim 1 whereby the compound has the structure

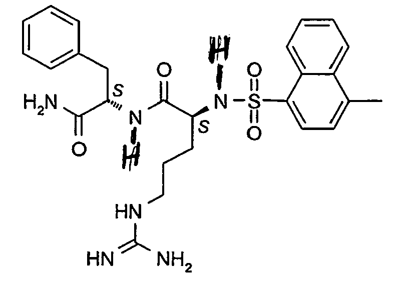

3. A compound of claim 1 whereby the compound has the structure

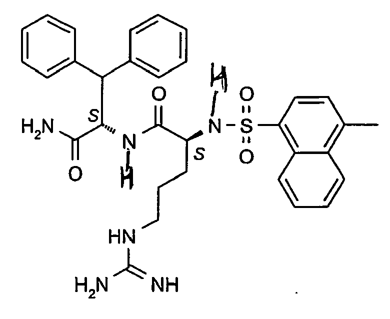

4. A compound of claim 1 whereby the compound has the structure

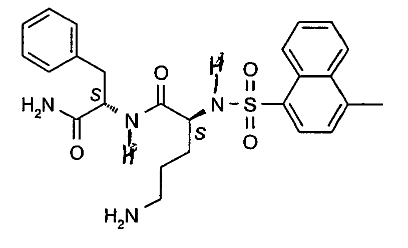

5. A compound of claim 1 whereby the compound has the structure

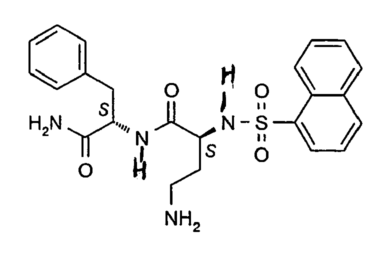

6. A compound of claim 1 whereby the compound has the structure

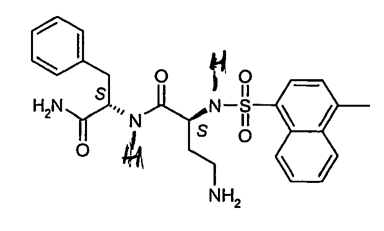

7. A compound of claim 1 whereby the compound has the structure

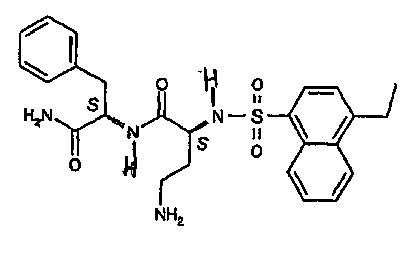

8. A compound of claim 1 whereby the compound has the structure

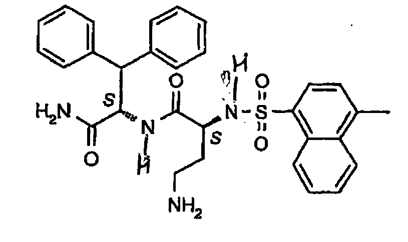

9. A compound of claim 1 whereby the compound has the structure

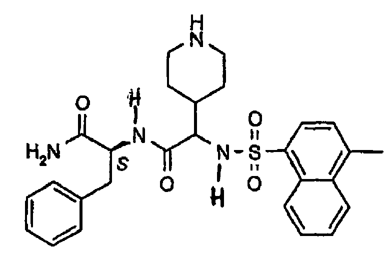

10. A pharmaceutical composition comprising of a compound according to any of claims 1 to 9 as an active ingredient together with a pharmaceutically acceptable diluent, carrier and/or excipient.

11. The use of a compound of any of claims 1 to 9, or a pharmaceutically acceptable salt thereof, for the preparation of a medicament for treating a disease or condition in mammals consisting of affective disorders or neurodegenerative diseases.

12. The use of a compound of any of claims 1 to 9, or a pharmaceutically acceptable salt thereof, for the preparation of a medicament for treating restenosis, age-related macular degeneration, vascular proliferation associated with angioplasty or vascular proliferation associated with AV shunts.

13. The use of a compound of any of claims 1 to 9, or a pharmaceutically acceptable salt thereof, for the preparation of a medicament for treating high intraocular pressure, deep ocular infections, glaucoma, stromal keratitis, iritis, retinitis, cataract, conjunctivitis, corneal graft rejection, retrolenteral fibroplasias, Osler-Webber-syndrome or rubeosis.

14. The use of a compound of any of claims 1 to 9, or a pharmaceutically acceptable salt thereof, for the preparation of a medicament for preventing or treating diabetic retinopathy, diabetic nephropathy, diabetic neuropathy, Doan syndrome and orthostatic hypertension.

15. The use of a compound of any of claims 1 to 9, or a pharmaceutically acceptable salt thereof, for the preparation of a medicament for treating tumors in mammals.

16. The use of a compund of any of claims 1 to 9, or a pharmaceutically acceptable salt thereof, for the preparation of a medicament for treating a disease or condition in mammals connected to wound healing, peptic ulcers, psoriasis, rheumatoid arthritis of Crohn's disease.

17. The use of a compound of any of claims 1 to 9, or a pharmaceutically acceptable salt thereof, for the preparation of a medicament for targeting tumors with SSTR1 and/or SSTR4 receptors using a compound of the invention conjugated with anti-cancer drugs directly or using a suitable spacer.

18. The use of a compound of any of claims 1 to 9, or a pharmaceutically acceptable salt thereof, for the preparation of an imaging agent for the medical imaging of healthy or diseased tissues and/or organs possessing SSTR4 and/or SSTR1 receptors.

## Patentansprüche

1. Verbindung der Formel I 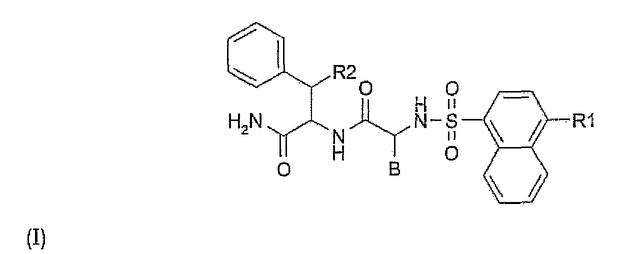 oder ein pharmazeutisch verträgliches Salz davon,
worin R1 ein H-Atom, eine Methyl- oder Ethylgruppe ist, R2 ein H-Atom oder eine Phenylgruppe ist und
B
1) -(CH₂)₃NHC(NH)NH₂,
2) -(CH₂)₃NH₂,
3) -(CH₂)₂NH₂ oder
4) 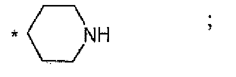
ist,
wobei der Stern (*) den Anbindungspunkt angibt,
mit der Maßgabe, dass, wenn B -(CH₂)₃NHC(NH)NH₂ ist, sodann R1 kein Wasserstoffatom ist.

2. Verbindung nach Anspruch 1, wobei die Verbindung die Struktur 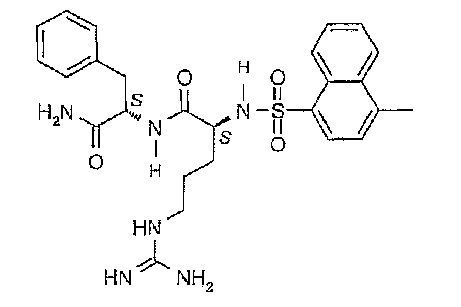 aufweist.

3. Verbindung nach Anspruch 1, wobei die Verbindung die Struktur 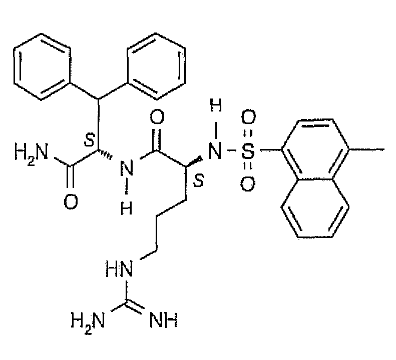 aufeist.

4. Verbindung nach Anspruch 1, wobei die Verbindung die Struktur 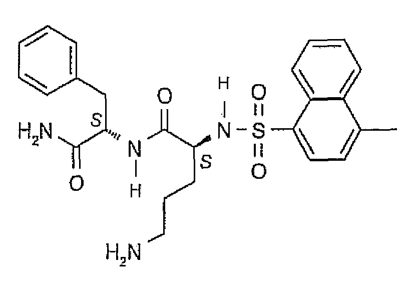 aufweist.

5. Verbindung nach Anspruch 1, wobei die Verbindung die Struktur 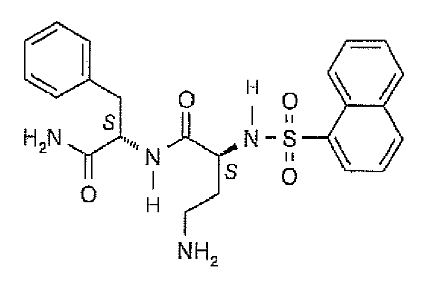 aufweist.

6. Verbindung nach Anspruch 1, wobei die Verbindung die Struktur 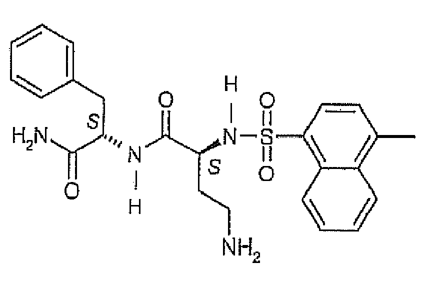 aufweist.

7. Verbindung nach Anspruch 1, wobei die Verbindung die Struktur 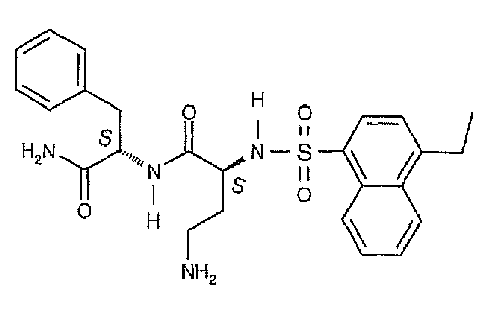 aufweist.

8. Verbindung nach Anspruch 1, wobei die Verbindung die Struktur 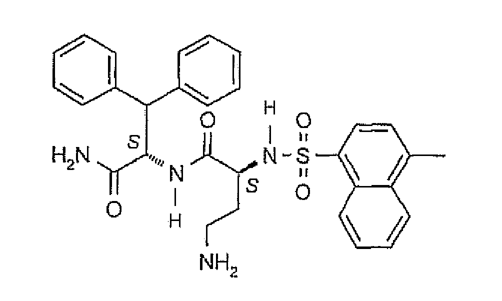 aufweist.

9. Verbindung nach Anspruch 1, wobei die Verbindung die Struktur 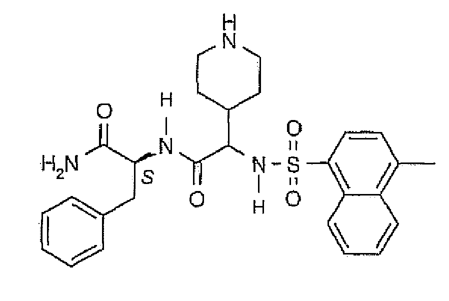 aufweist.

10. Pharmazeutische Zusammensetzung, die eine Verbindung gemäß einem der Ansprüche 1 bis 9 als einen wirksamen Bestandteil zusammen mit einem pharmazeutisch verträglichen Verdünnungsmittel, Träger und/order Exzipiens umfasst.

11. Verwendung einer Verbindung nach einem der Ansprüche 1 bis 9 oder eines pharmazeutisch verträglichen Salzes davon zur Herstellung eines Medikaments zum Behandeln einer Erkrankung oder eines Zustands in Säugern, bestehend aus affektiven Erkrankungen oder neurodegenerativen Erkrankungen.

12. Verwendung einer Verbindung nach einem der Ansprüche 1 bis 9 oder eines pharmazeutisch verträglichen Salzes davon zur Herstellung eines Medikaments zum Behandeln von Restenose, altersbedingter Makuladegeneration, mit Angioplastie assoziierter vaskulärer Proliferation oder mit AV-Shunts assoziierter vaskuläre Proliferation.

13. Verwendung einer Verbindung nach einem der Ansprüche 1 bis 9 oder eines pharmazeutisch verträglichen Salzes davon zur Herstellung eines Medikaments zum Behandeln von intraokularem Hochdruck, tiefen okularen Infektionen, Glaukom, Stromakeratitis, Iritis, Retinitis, Katarakt, Konjunktivitis, Hornhauttransplantatabstoßung, retrolentale Fibroplasien, Osler-Weber-Syndrom oder Rubeose.

14. Verwendung einer Verbindung nach einem der Ansprüche 1 bis 9 oder eines pharmazeutisch verträglichen Salzes davon zur Herstellung eines Medikaments zum Verhindern oder Behandeln von diabetischer Retinopathie, diabetischer Nephropathie, diabetischer Neuropathie, Doan-Syndrom und orthostatischer Hypertonie.

15. Verwendung einer Verbindung nach einem der Ansprüche 1 bis 9 oder eines pharmazeutisch verträglichen Salzes davon zur Herstellung eines Medikaments zum Behandeln von Tumoren in Säugern.

16. Verwendung einer Verbindung nach einem der Ansprüche 1 bis 9 oder eines pharmazeutisch verträglichen Salzes davon zur Herstellung eines Medikaments zum Behandeln einer Erkrankung oder eines Zustands in Säugern, die/der mit Wundheilung, Magen- und Zwölffingerdarmgeschwüren, Schuppenflechte, rheumatoider Arthritis oder Morbus Crohn verbunden ist.

17. Verwendung einer Verbindung nach einem der Ansprüche 1 bis 9 oder eines pharmazeutisch verträglichen Salzes davon zur Herstellung eines Medikaments zum Abzielen auf Tumore mit SSTR1- und/oder SSTR4-Rezeptoren unter Verwendung einer erfindungsgemäßen Verbindung, die mit Antikrebsarzneimitteln direkt oder unter Verwendung eines geeigneten Spacers konjugiert ist.

18. Verwendung einer Verbindung nach einem der Ansprüche 1 bis 9 oder eines pharmazeutisch verträglichen Salzes davon zur Herstellung eines Bildgebungsmittels zum medizinischen Bildgeben von gesunden oder erkrankten Geweben und/oder Organen, die SSTR4- und/oder SSTR1-Rezeptoren aufweisen.

## Revendications

1. Composé de formule I 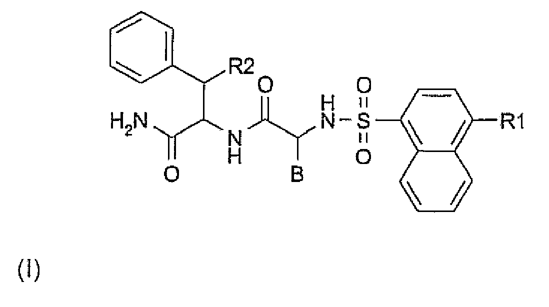 ou sel dérivé de celui-ci pharmaceutiquement acceptable ;
dans lequel le R1 est un atome d'hydrogène H, un groupement méthyle ou éthyle ; R2 est un atome d'hydrogène H ou un groupement phényle et B est le radical
1) -(CH₂)₃NHC(NH)NH₂,
2) -(CH₂)₃NH₂,
3) -(CH₂)₂NH₂ ou
4) 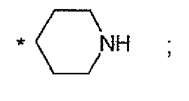
dans lequel l'astérisque (*) indique le point de liaison ;
à condition que, lorsque B est -(CH₂)₃NHC(NH)NH₂, alors R1 n'est pas un atome d'hydrogène.

2. Composé de la revendication 1, ce composé ayant la structure

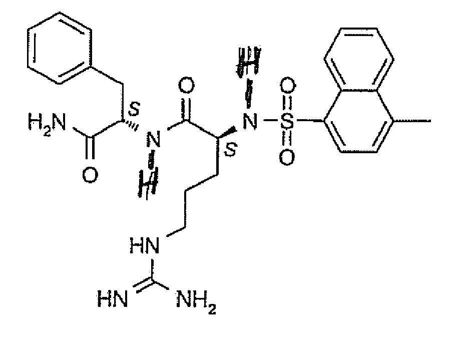

3. Composé de la revendication 1, ce composé ayant la structure

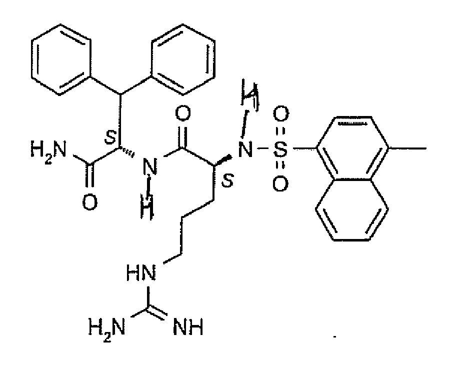

4. Composé de la revendication 1, ce composé ayant la structure

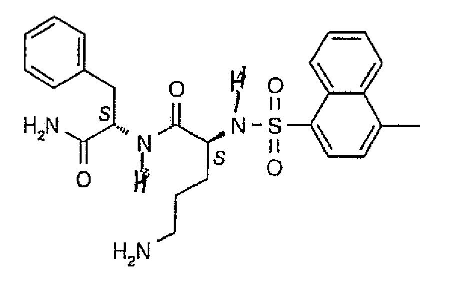

5. Composé de la revendication 1, ce composé ayant la structure

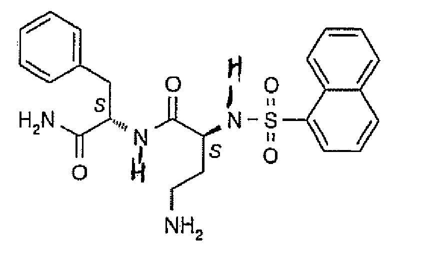

6. Composé de la revendication 1, ce composé ayant la structure

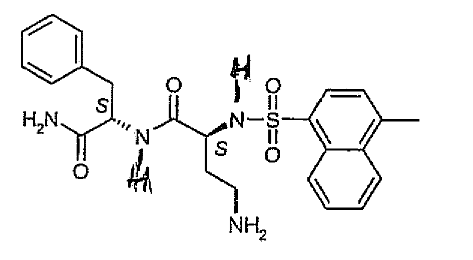

7. Composé de la revendication 1, ce composé ayant la structure

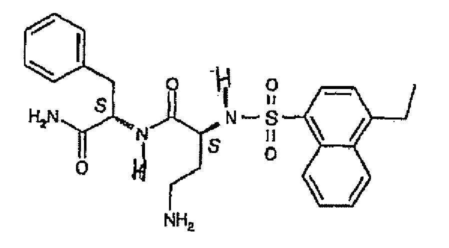

8. Composé de la revendication 1, ce composé ayant la structure

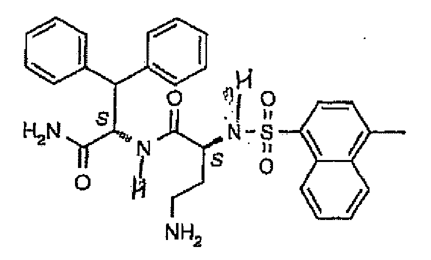

9. Composé de la revendication 1, ce composé ayant la structure

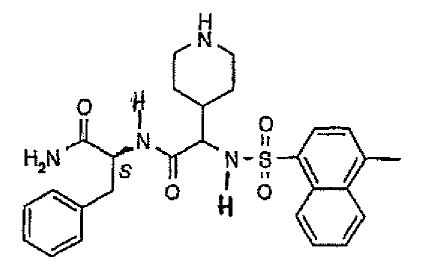

10. Composition pharmaceutique comprenant un composé selon l'une quelconque des revendications 1 à 9 comme substance active ainsi qu'un diluant, un vecteur et/ou un excipient pharmaceutiquement acceptable.

11. Utilisation d'un composé de l'une quelconque des revendications 1 à 9, ou d'un sel dérivé de celui-ci pharmaceutiquement acceptable, pour la préparation d'un médicament pour le traitement d'une maladie ou d'un état chez les mammifères consistant en des désordres affectifs ou en des maladies neurodégénératives.

12. Utilisation d'un composé de l'une quelconque des revendications 1 à 9, ou d'un sel dérivé de celui-ci pharmaceutiquement acceptable, pour la préparation d'un médicament pour traiter la resténose, la dégénérescence maculaire due à l'âge, la prolifération vasculaire associée à l'angioplastie ou la prolifération vasculaire associée aux dérivations artérioveineuses.

13. Utilisation d'un composé de l'une quelconque des revendications 1 à 9, ou d'un sel dérivé de celui-ci pharmaceutiquement acceptable, pour la préparation d'un médicament pour traiter une pression intraoculaire élevée, des infections oculaires profondes, un glaucome, une kératopathie de strome, une iritis, une rétinite, la cataracte, la conjonctivite, le rejet de greffe cornéen, des fibroplasies retrolenticulaires, le syndrome d'Osler-Webber ou la rubéose.

14. Utilisation d'un composé de l'une quelconque des revendications 1 à 9, ou un sel dérivé de celui-ci pharmaceutiquement acceptable, pour la préparation d'un médicament pour empêcher ou traiter une rétinopathie diabétique, une néphropathie diabétique, une neuropathie diabétique, le syndrome de Doan et l'hypertension orthostatique.

15. Utilisation d'un composé de l'une quelconque des revendications 1 à 9, ou d'un sel dérivé de celui-ci pharmaceutiquement acceptable, pour la préparation d'un médicament pour traiter des tumeurs chez les mammifères.

16. Utilisation d'un composé de l'une quelconque des revendications 1 à 9, ou d'un sel dérivé de celui-ci pharmaceutiquement acceptable, pour la préparation d'un médicament pour traiter une maladie ou un état chez les mammifères lié(e) à la cicatrisation de plaies, aux ulcères peptiques, aux psoriasis, à l'arthrite rhumatoïde de la maladie de Crohn.

17. Utilisation d'un composé de l'une quelconque des revendications 1 à 9, ou d'un sel dérivé de celui-ci, pour la préparation d'un médicament pour viser des tumeurs avec des récepteurs SSTR1 et/ou SSTR4 en utilisant un composé de l'invention associé à des médicaments anticancéreux de manière directe ou en utilisant un tube d'espacement approprié.

18. Utilisation d'un composé de l'une quelconque des revendications 1 à 9, ou d'un sel dérivé de celui-ci, pour la préparation d'un agent d'imagerie pour l'imagerie médicale de tissus et/ou d'organes sains ou malades possédant des récepteurs SSTR4 et/ou SSTR1.
